# EUROPÄISCHE PATENTANMELDUNG

(11) **EP 0 542 083 A1**
(43) Veröffentlichungstag der Anmeldung: **19.05.1993**
(21) Anmeldenummer: 92118740.7
(22) Anmeldetag: 02.11.1992
(51) Int. Cl.: C07C 253/14, C09B 47/067

(54) **Verfahren zur Herstellung von 1,2,3-Tricyanobenzol, aus 1,2,3-Tricyanobenzol erhältliche Phthalocyanine und ihre Verwendung als Pigmente**

(30) Priorität: 13.11.1991 DE 4137270
(71) Anmelder: BAYER AG, 51368 Leverkusen (DE)
(72) Erfinder: Beck, Gunther, Dr., W-5090 Leverkusen 1 (DE); Kaletta, Bernd, Dr., W-5090 Leverkusen 1 (DE)

(57) **Zusammenfassung**

Aus Fluorbenzonitrilen läßt sich durch Umsetzung mit Alkalicyanid in guter Ausbeute 1,2,3-Tricyanobenzol herstellen. Dieses kann durch Tetramisierung, gegebenenfalls in Gegenwart von Metallen oder Metallverbindungen zu metallfreien Tetracyanophthalocyaninen oder zu den entsprechenden Metallkomplexen umgesetzt werden. Diese stellen ausgezeichnete organische Pigmente dar.

## Beschreibung

Die Erfindung betrifft ein Verfahren zur Herstellung von 1,2,3-Tricyanobenzol aus Fluorbenzonitrilen durch Umsetzung mit Alkalicyaniden, aus 1,2,3-Tricyanobenzol erhältliche Tetracyanophthalocyanine und die Verwendung dieser Phthalocyanine als Farbpigmente.

1,2,3-Tricyanobenzol und verschiedene Verfahren zu seiner Herstellung sind bekannt. Laut J. Chem. Soc., Chem. Commun. 972 (1976) entsteht bei der Umsetzung von 1,3-Dicyano-2-nitrobenzol mit Kaliumcyanid in Dimethylsulfoxid oder Dimethylformamid bei 100°C ein Reaktionsgemisch, das neben 65 - 75 % 2,6-Dicyanophenol und ca. 10 % schwarzem, harzartigem Material ca. 15 % 1,2,3-Tricyanobenzol enthalten soll. Die genannte Publikation enthält keinerlei weitergehende experimentelle Angaben.

Bei der Umsetzung von Cyanacetylen mit [2,2]-Paracyclophan bei 160°C wurde als Nebenprodukt in 15%iger Ausbeute ein 1:4-Gemisch von 1,2,3- und 1,2,4-Tricyanobenzol (entsprechend einer Ausbeute von 3 % 1,2,3-Tricyanobenzol, bezogen auf Cyanacetylen) isoliert; Chem. Ber. 123 (1990), 2015.

Gemäß J. Org. Chem. 55 (1990), 5601 ist auch die Herstellung von 1,2,3-Tricyanobenzol aus 1,2,3-Benzoltricarbonsäure (z.B. erhältlich durch Kaliumpermanganat-Abbau von Naphthalsäureanhydrid) in 3-stufiger Reaktion über das Säurechlorid und das Säureamid in einer Gesamtausbeute von knapp 12 % der Theorie möglich.

Aufgabe der Erfindung war daher unter anderem das Bereitstellen eines einfachen und wirtschaftlichen Verfahrens zur Herstellung von 1,2,3-Tricyanobenzol.

Überraschenderweise wurde gefunden, daß 1,2,3-Tricyanobenzol durch Umsetzung von Fluorcyanobenzolen mit Alkalicyanid in guter Ausbeute zugänglich ist.

Gegenstand der Erfindung ist daher ein Verfahren zur Herstellung von 1,2,3-Tricyanobenzol durch Umsetzung von Verbindungen der Formel 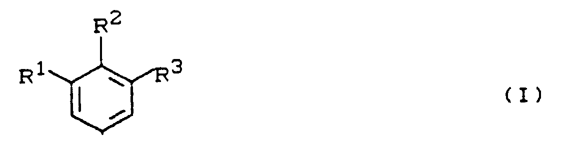
worin R¹, R², R³
unabhängig voneinander F oder CN bedeuten mit der Maßgabe, daß mindestens 1 F- und mindestens 1 CN-Rest vorhanden sind,
mit Alkalicyanid.

Die Ausgangsverbindungen (I) umfassen 2,3- und 2,6-Difluorbenzonitril, 1,2-Dicyano-3-fluorbenzol und 1,3-Dicyano-2-fluorbenzol. Bevorzugter Ausgangsstoff ist das in industriellem Maßstab hergestellte 2,6-Difluorbenzonitril.

Als Alkalicyanide werden vorzugsweie die Cyanide des Natriums oder Kaliums verwendet.

Das erfindungsgemäße Verfahren wird vorzugsweise in organischen Verdünnungsmitteln ausgeführt; als solche eignen sich insbesondere aprotische dipolare organische Lösungsmittel, z.B. N,N-Di-C₁-C₄-alkylamide aliphatischer C₁-C₆-Carbonsäuren, wie N,N-Dimethylformamid, N,N-Diethylformamid, N-Methylpyrrolidinon, N-Methylcaprolactam; aliphatische Sulfoxide wie Dimethylsulfoxid, aliphatische Sulfone wie Dimethylsulfon und Tetramethylensulfon; ferner Tetramethylharnstoff, N,N'-Dimethyl-1,3-imidazolidin-2-on und Hexamethylphosphorsäuretrisamid. Besonders bevorzugt sind N,N-Dimethylformamid und N-Methylpyrrolidinon.

Die Menge Verdünnungsmittel wählt man im allgemeinen so, daß pro Gramm Ausgangsprodukt (I) 1 bis 100, vorzugsweise 5 bis 20 ml Verdünnungsmittel eingesetzt werden.

Zur vollständigen Umsetzung eines Mols Dicyanofluorbenzol sind formal 1 Mol Alkalicyanid, zur Umsetzung eines Mols Difluorbenzonitril 2 Mol Alkalicyanid erforderlich. Es kann jedoch günstig sein, mit einem Unterschuß Alkalicyanid zu arbeiten oder die Reaktion vorzeitig abzubrechen, weil die nicht umgesetzten Ausgangsprodukte, insbesondere das 2,6-Difluorbenzonitril, aufgrund ihrer höheren Flüchtigkeit aus dem Reaktionsgemisch besonders leicht abtrennbar sind. In der Regel wird man 50 bis 120, vorzugsweise 50 bis 100, insbesondere 50 bis 99 % Mol Alkalicyanid pro val Fluorid einsetzen.

Das erfindungsgemäße Verfahren kann bei Temperaturen von -50°C bis +100°C, vorzugsweise von -20°C bis +60°C, besonders bevorzugt von 0°C bis +30°C ausgeführt werden, wobei milde Bedingungen das Entstehen unerwünschter Produkte weitgehend vermeiden.

Die Reaktion wird im allgemeinen bei Normaldruck vorgenommen.

Das erfindungsgemäße Verfahren wird vorzugsweise so ausgeführt, daß man die Lösung des Ausgangsprodukts (I) in einem wasserfreien Lösungsmittel unter Feuchtigkeitsausschluß und unter Rühren mit dem Alkalicyanid versetzt und z.B. 10 bis 100 Stunden kräftig weiterrührt. Nach beendeter (oder vorzeitig abgebrochener) Umsetzung wird die Reaktionsmischung dann in überschüssiges (z.B. das zwei- bis 10-fache Volumen) Eiswasser gegeben. Man kann auch das Lösungsmittel vor dem Verrühren des Reaktionsgemisches in Wasser ganz oder teilweise abziehen, wobei gegebenenfalls vorhandene Restmengen unumgesetzten Alkalicyanids vorher zweckmäßigerweise durch Filtration entfernt werden. Das beim Verrühren mit Wasser ausgefallene 1,2,3-Tricyanobenzol wird durch Filtration isoliert und gegebenenfalls nachgereinigt, beispielsweise durch Vakuumsublimation und/oder Umkristallisation.

Weiterhin wurde gefunden, daß sich 1,2,3-Tricyanobenzol hervorragend als Ausgangsmaterial für die Herstellung von neuen Tetracyano-phthalocyaninen und deren Metallkomplexen eignet.

Weiterer Gegenstand der Erfindung sind also Verbindungen der Formel 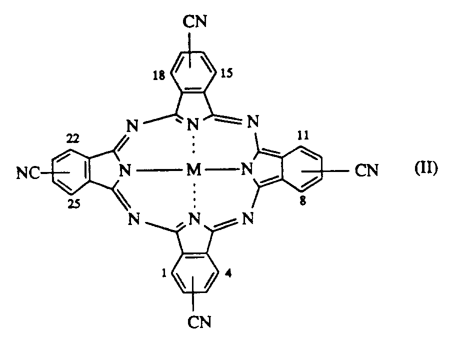
worin
die Nitrilgruppen jeweils in einer der beiden möglichen o-Positionen stehen (wobei unter "o-Position" die 1-, 4-, 8-, 11-, 15-, 18-, 22- und 25-Stellungen im Phthalocyanin II verstanden werden) und
M 2 Wasserstoffatome, Magnesium, Calcium, Kupfer, Eisen, Cobalt, Nickel, Zink, Zinn, Vanadiumoxy, Titanoxy, Chromoxy oder Mangan bedeutet.

Die Phthalocyanine (II) können analog bekannten Verfahren, wie sie beispielsweise bei
K. Venkataraman, "The Chemistry of Synthetic Dyes", Vol. II, Academic Press, Inc., New York, 1952, S. 1118 - 1142,
oder
N.M. Bigelow und M.A. Perkins in Lubs (Hrsg.), "The Chemistry of Synthetic Dyes und Pigments", Reinhold Publishing Corp., New York, 1955, S. 577-606,
und in der dort zitierten Literatur beschrieben sind, hergestellt werden. So lassen sich die Metallkomplexe (II) beispielsweise 1) durch direkte Reaktion von 1,2,3-Tricyanobenzol mit Metallen, Metallsalzen, -oxiden oder -hydroxiden, gegebenenfalls in Gegenwart von Basen, 2) durch Metalladdition an metallfreie Phthalocyanine (II) und 3) durch Metallaustausch mit anderen Metallophthalocyaninen (II) unter geeigneten Bedingungen herstellen. Metallfreie Phthalocyanine (II) sind 1) durch direkte Tetramerisierung von 1,2,3-Tricyanobenzol in Gegenwart von Basen und 2) durch Entfernen des Zentralatoms aus den entsprechenden Metallophthalocyaninen (II) zugänglich. Die Herstellung muß nicht, kann aber in inerten organischen Lösungsmitteln erfolgen.

Zu den geeigneten Metallsalzen, -oxiden und -hydroxiden zur Herstellung der Metallkomplexe (II) zählen z.B. Magnesiumchlorid, -bromid, -acetat, -oxid, -hydroxid, Calciumchlorid und -acetat, Kupfer(I)-chlorid, -bromid und -cyanid, Kupfer(II)-chlorid, -acetat und -nitrat, Eisen(II)-chlorid, Eisen(III)-chlorid und -nitrat, Cobaltchlorid, -bromid und -acetat, Nickelchlorid, -bromid, -acetat und -oxid, Zinkchlorid und -acetat, Zinnchlorid und -acetat, Vanadium(III)- und -(IV)-chlorid, Vanadium-(V)-oxychlorid, Titan(III)- und -(IV)-chlorid, Chrom-(III)-chlorid, -fluorid und -oxid, Mangan(II)-chlorid und -acetat.

Sie werden in der Regel in Mengen von 0,5 bis 10, vorzugsweise 1 bis 2 Mol pro Mol metallfreies Phthalocyanin (II) eingesetzt.

Geeignete Basen zur Herstellung der metallfreien und metallhaltigen Phthalocyanine (II) umfassen beispielsweise tertiäre Amine wie 1.4-Diazabicyclo[2.2.2]-octan, 1,8-Diazabicyclo[5.4.0]-undec-7-en, 1,5-Diazabicyclo[4.3.0]-non-5-en, Natrium-, Kalium- und Lithiumalkoholate einwertiger C₁-C₁₂-Alkohole wie Methanol, Ethanol, Isopropanol, Butanol, Pentanol usw. oder zweiwertiger C₂-C₁₂-Alkohole wie Ethylenglykol u.a.

Geeignete organische Lösungsmittel zur Herstellung der Phthalocyanine (II) sind z.B. die oben genannten Alkohole selbst, aprotische dipolare organische Lösungsmittel, z.B. N,N-Di-C₁-C₄-alkylamide aliphatischer C₁-C₆-Carbonsäuren, wie N,N-Dimethylformamid, N,N-Diethylformamid, N-Methylpyrrolidinon, N-Methylcaprolactam; aliphatische Sulfoxide wie Dimethylsulfoxid, aliphatische Sulfone wie Dimethylsulfon und Tetramethylensulfon; ferner Tetramethylharnstoff, N,N'-Dimethyl-1,3-imidazolidin-2-on und Hexamethylphosphorsäuretrisamid.

Sie werden im allgemeinen in Mengen von 1 bis 50, vorzugsweise 5 bis 20 Gew.-%, bezogen auf die Summe der Ausgangskomponenten, eingesetzt.

Die Herstellung kann bei Temperaturen von 100 bis 300, vorzugsweise 120 bis 200°C erfolgen. Die Reaktion ist beendet, wenn im Dünnschichtchromatogramm kein Edukt mehr nachweisbar ist.

Die erfindungsgemäßen Tetracyanophthalocyanine (II) können zur Herstellung von sehr echt pigmentierten Systemen, wie Mischungen mit anderen Stoffen, Zubereitungen, Anstrichmitteln, Druckfarben, gefärbtem Papier und gefärbten makromolekularen Stoffen verwendet werden. Unter Mischungen mit anderen Stoffen können z.B. solche mit anorganischen Weißpigmenten wie Titandioxid (Rutil) verstanden werden. Zubereitungen sind z.B. Flushpasten mit organischen Flüssigkeiten und gegebenenfalls Konservierungsmitteln. Die Bezeichnung Anstrichmittel steht z.B. für physikalisch oder oxidativ trocknende Lacke, Einbrennlacke, Reaktionslacke, Zweikomponentenlacke, Dispersionsfarben für wetterfeste Überzüge und Leimfarben. Unter Druckfarben sind solche für den Papier-, Textil- und Blechdruck zu verstehen. Insbesondere eignen sich die erfindungsgemäßen Phthalocyanine (II) zum Pigmentieren von makromolekularen organischen Stoffen.

Die makromolekularen Stoffe können natürlichen Ursprungs sein wie Kautschuk, durch chemische Modifikation erhalten werden wie Acetylcellulose, Cellulosebutyrat oder Viskose oder synthetisch erzeugt werden wie Polymerisate, Polyadditionsprodukte und Polykondensate. Genannt seien thermoplastische Polymere wie Polyvinylchlorid, Polyvinylacetat, Polyvinylpropionat, Superpolyamide, Polymerisate und Mischpolymerisate aus Acrylestern und/oder Methacrylestern, Acrylamid, Butadien, Styrol sowie Polyurethane und Polycarbonate. Die mit den erfindungsgemäßen Phthalocyaninen (II) pigmentierten Stoffe können in beliebiger Form vorliegen.

Wegen ihrer hohen Transparenz und Wetterechtheit eignen sich die erfindungsgemäßen Phthalocyanine (II) besonders zum Einsatz in Automobillacken, insbesondere für Metalliclackierungen. Sie können auch als Laserfarbstoffe dienen.

Die erfindungsgemäßen Phthalocyanine (II) sind ausgezeichnet wasserecht, ölecht, säureecht, kalkecht, alkaliecht, lösungsmittelecht, überlackierecht, überspritzecht, sublimierecht, hitzebeständig, vulkanisierbeständig, sehr ergiebig, in plastischen Massen gut verarbeitbar und insbesondere ausgezeichnet wetter-, licht- und migrationsecht.

Weiterer Gegenstand der Erfindung ist also schließlich die Verwendung der Phthalocyanine (II) als Farbpigmente.

Die Prozentangaben der nachfolgenden Beispiele beziehen sich auf das Gewicht; Teile sind Gewichtsteile.

### Beispiel 1

### Herstellung von 1,2,3-Tricyanobenzol

Die Lösung von 150 g (1,08 Mol) 2,6-Difluorbenzonitril in 1500 ml trockenem Dimethylformamid wird unter Rühren und unter Feuchtigkeitsausschluß bei Raumtemperatur mit 79,4 g (1,62 Mol) Natriumcyanid versetzt. Das Reaktionsgemisch wird 24 Stunden bei Raumtemperatur kräftig gerührt.

Anschließend wird es unter Rühren in 7,5 l Eiswasser gegossen. Danach wird der entstandene Niederschlag abfiltriert, mit Wasser gewaschen und bis zur Gewichtskonstanz getrocknet. Das Rohprodukt (108,5 g) wird anschließend bei etwa 0,1 mbar bis zu einer Badtemperatur von 200°C sublimiert. Ausbeute 100,5 g 1,2,3-Tricyanobenzol entsprechend 81,1 % der Theorie, bezogen auf das im Unterschuß eingesetzte Natriumcyanid. Schmelzpunkt 177 - 178°C (aus Toluol oder 1,2-Dichlorethan). IR (KBr): 3097, 3078, 3072, 2241, 1580, 1459, 1243, 828, 738 [cm⁻¹].

### Beispiel 2

### Herstellung von Tetracyanophthalocyanin

15,3 Teile 1,2,3-Tricyanobenzol werden in 150 Teilen Amylalkohol gelöst. Dann werden 9 Teile einer 30%igen Natriummethylat-Lösung in Methanol zugesetzt und es wird 15 Stunden zum Rückfluß erhitzt. Das gebildete Dinatriumsalz wird kalt abgesaugt, in 100 Teilen DMF aufgenommen und mit 20 Teilen Essigsäure versetzt. Es wird 1 Stunde bei Raumtemperatur nachgerührt; das Pigment wird abgesaugt, mit Methanol gewaschen und im Trockenschrank bei 50°C getrocknet. Es werden 12,8 g petrolfarbenes Tetracyanophthalocyanin erhalten.

### Beispiel 3

### Herstellung von Cu-Tetracyanophthalocyanin

18 Teile 1,2,3-Tricyanobenzol, 6 Teile Kupfer(II)acetat-Monohydrat und 500 Teile N-Methylpyrolidon werden 1 Stunde bei 100°C gerührt; anschließend wird 15 Stunden zum Rückfluß erhitzt. Das Produkt wird kalt abgesaugt, mit Methanol gewaschen und im Trockenschrank bei 50°C getrocknet. Es werden 16,2 g blaues Kupfer-tetracyanophthalocyanin erhalten. Eine der denkbaren Strukturen ist:

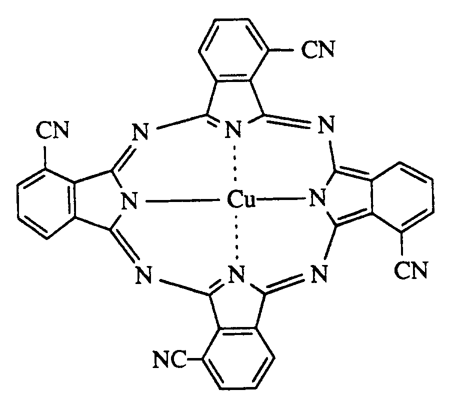

### Beispiel 4 - 7

### Herstellung weiterer Metallo-tetracyanophthalocyanine

Nach dem in Beispiel 3 genannten Verfahren wurden bei Einsatz der entsprechenden Metallacetate oder Metallchloride die entsprechenden Metallkomplexe (II) mit der in der Tabelle genannten Bedeutung für M und den zugehörigen Farbtönen erhalten:

| Beispiel | M = | Farbton |
|---|---|---|
| 4 | Ni | türkis |
| 5 | Co | blau |
| 6 | Ti=0 | blau |
| 7 | V=0 | blau |

### Anwendung 1

### Pigmentierte Einbrennlackierung

8 g feingemahlenes Pulver gemäß Beispiel 2 werden in 92 g eines Einbrennlackes folgender Zusammensetzung dispergiert:
33 % Alkydharz
15 % Melamin/-Formaldehyd-Harz
5 % Glykolmonomethylether
34 % Xylol
13 % Butanol
Als Alkydharze kommen Produkte auf Basis synthetischer und pflanzlicher Fettsäure wie Kokosöl, Rizinusöl, Rizinenöl, Leinöl und andere in Frage. Anstelle von Melamin/Formaldehyd-Harzen können Harnstoff/Formaldehyd-Harze verwendet werden.

Nach erfolgter Dispergierung wird der pigmentierte Lack auf Papier-, Glas-, Kunststoff- oder Metall-Folien aufgetragen und 30 Minuten bei 130°C eingebrannt. Die Lackierungen besitzen sehr gute Licht- und Wetterbeständigkeit sowie gute Überlackierechtheit.

### Anwendung 2

### Pigmentierte Thermoplaste

0,2 g Pigment nach Beispiel 2 werden mit 100 g Polyethylen-, Polypropylen- oder Polystyrolgranulat gemischt. Die Mischung kann entweder bei 220 bis 280°C direkt in einer Spritzgußmaschine verspritzt oder in einer Strangpresse zu gefärbten Stäben beziehungsweise auf dem Mischwalzwerk zu gefärbten Fellen verarbeitet werden. Die Stäbe beziehungsweise Felle werden gegebenenfalls granuliert und in einer Spritzgußmaschine verspritzt.

Die blaugrünen Formlinge besitzen sehr gute Licht- und Migrationsechtheit. In ähnlicher Weise können bei 280 - 300°C, gegebenenfalls unter Stickstoffatmosphäre, synthetische Polyamide aus Caprolactam oder aus Adipinsäure und Hexamethylendiamin oder Polyethylenterephthalat gefärbt werden.

### Anwendung 3

### Pigmentierte Druckfarbe

Mit einer Druckfarbe, hergestellt durch Anreiben von 35 g Pigment nach Beispiel 2 und 65 g Leinöl und Zugabe von 1 g Siccativ (Co-Naphthenat, 50%ig in Testbenzin) werden blaugrüne Offset-Drucke hoher Brillanz und Farbstärke und sehr gute Licht- und Lackierechtheiten erhalten. Verwendung dieser Druckfarbe in Buch-, Licht-, Stein- oder Stahlstichdruck führt zu blaugrünen Drucken ähnlicher Echtheiten. Verwendet man das Pigment zur Färbung von Blechdruck oder Tiefdrucken oder niedrigviskosen Drucktinten, erhält man blaugrüne Drucke ähnlicher Echtheiten.

## Patentansprüche

1. Verfahren zur Herstellung von 1,2,3-Tricyanobenzol durch Umsetzung von Verbindungen der Formel 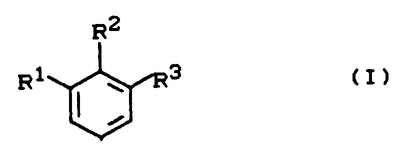 worin R¹, R², R³
unabhängig voneinander F oder CN bedeuten mit der Maßgabe, daß mindestens 1 F- und mindestens 1 CN-Rest vorhanden sind, mit Alkalicyanid.

2. Verbindungen der Formel 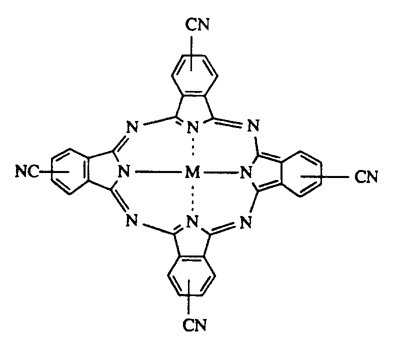 worin
die Nitrilgruppen jeweils in einer der beiden möglichen o-Positionen stehen und
M 2 Wasserstoffatome, Magnesium, Calcium, Kupfer, Eisen, Cobalt, Nickel, Zink, Zinn, Vanadiumoxy, Titanoxy, Chromoxy oder Manganoxy bedeutet.

3. Verfahren zur Herstellung von Verbindungen II nach Anspruch 2 durch Tetramerisierung von 1,2,3-Tricyanobenzol, gegebenenfalls in Gegenwart von Metallen, Metallsalzen, -oxiden oder -hydroxiden aus der unter M in Anspruch 2 aufgeführten Reihe.

4. Verwendung der Verbindungen II nach Anspruch 2 als Farbpigmente.
